# EUROPÄISCHE PATENTSCHRIFT

(11) **EP 4 368 227 B1**
(45) Veröffentlichungstag und Bekanntmachung des Hinweises auf die Patenterteilung: **26.02.2025**
(21) Anmeldenummer: 22206494.1
(22) Anmeldetag: 09.11.2022
(51) Int. Cl.: A61M 11/00, A61M 15/00, A61M 15/08, B05B 1/34, B05B 11/00, B05B 11/10

(54) **FLÜSSIGKEITSSPENDER FÜR NASALE APPLIKATION UND PUMPEINRICHTUNG**
NASAL FLUID DISPENSER AND PUMPING DEVICE
DISTRIBUTEUR DE LIQUIDE POUR APPLICATION NASALE ET DISPOSITIF DE POMPAGE

(43) Veröffentlichungstag der Anmeldung: 15.05.2024
(73) Patentinhaber: Aptar Radolfzell GmbH, 78315 Radolfzell (DE)
(72) Erfinder: Greiner-Perth, Jürgen, 78244 Gottmadingen (DE); Ritsche, Stefan, 78253 Eigeltingen (DE)
(74) Vertreter: Witte, Weller und Partner Patentanwälte mbB Stuttgart

(56) Entgegenhaltungen:
- EP-A1- 3 412 325
- EP-A2- 2 236 213
- DE-A1- 102011 082 420
- DE-B3- 102009 037 164

## Beschreibung

Die Erfindung betrifft einen Nasalspender für die nasale Applikation von zerstäubten Flüssigkeiten sowie ein insbesondere für einen solchen Nasalspender geeignete Pumpeinrichtung.

Gattungsgemäße Nasalspender finden Anwendung, um Flüssigkeit in zerstäubter Form nasal über die Nasenlöcher eines Patienten zu applizieren, insbesondere pharmazeutische Flüssigkeiten zur Linderung von Atembeschwerden bei Erkältungssymptomen. Die Applikation der zerstäubten Flüssigkeit in Form eines konischen Sprühstrahls begünstigt die gewünschte Verteilung der Flüssigkeit in den Atemwegen.

Die Herstellung eines Sprühstrahls von zerstäubter Flüssigkeit kann bei Nasalspendern auf verschiedenen Wegen erfolgen, so beispielsweise durch eine Düsenplatte mit einer Vielzahl von Düsenöffnungen oder mittels einer Wirbelkammer, in der die Flüssigkeit vor dem Austrag in eine schnelle Wirbelbewegung gebracht wird, wobei eine Austragöffnung stirnseitig an der Wirbelkammer vorgesehen ist und die wirbelnde Flüssigkeit beim dortigen Austrag zu feinen Tröpfchen zerrissen wird. Die Verwendung von Nasalspendern, die Flüssigkeit in zerstäubter Form austragen, ist insbesondere bei Erwachsenen üblich. Bei Kindern wird die Handhabung von Nasentropfen jedoch häufig als einfacher wahrgenommen, auch weil Kinder mitunter den Sprühstoß mit zerstäubter Flüssigkeit als unangenehm empfinden. EP3412325 A1 betrifft einen Austragkopf für die nasale Applikation von pharmazeutischen Flüssigkeit aus einem Druckspeicher, welcher über ein Auslassventil mit einem Ventilstutzen verfügt, der gegen eine Federkraft zum Öffnen des Auslassventils kraftbeaufschlagbar ist.

### AUFGABE UND LÖSUNG

Aufgabe der Erfindung ist es, einen Nasalspender für die nasale Applikation von Flüssigkeit in zerstäubter Form bereitzustellen, dessen Austrag von Kindern als weniger unangenehm wahrgenommen wird.

Zur Lösung dieser Aufgabe wird ein Nasalspender für die nasale Applikation von Flüssigkeiten vorgeschlagen, insbesondere zur Verwendung bei Kindern, der in gattungsüblicher Weise über einen Flüssigkeitsspeicher zur Aufnahme von Flüssigkeit vor der Abgabe sowie über einen schlanken Nasalapplikator zur Einführung in die Nase und zur Abgabe der Flüssigkeit in einer Austragrichtung verfügt. Der Nasalapplikator ist vorzugsweise Teil einer gegenüber dem Flüssigkeitsspeicher linear in einer Betätigungsrichtung und insbesondere parallel zur Austragrichtung verlagerbaren Austrageinheit, durch deren Niederdrücken gegen die Kraft einer Rückstellfeder eine Pumpeinrichtung betätigt oder eine Ventileinrichtung geöffnet wird.

Der Nasalapplikator weist ein Außenbauteil und ein Innenbauteil auf. Das Außenbauteil bildet eine äußere und für den Nutzer sichtbare Außenfläche. Das Außenbauteil weist eine schlanke Applikatorspitze mit einer distalen Stirnwandung auf, die von einer Austragöffnung durchdrungen ist, welche zur Abgabe eines Sprühstrahls in Austragrichtung ausgebildet ist. Die Austragrichtung entspricht dabei vorzugsweise der Haupterstreckungsrichtung der schlanken Applikatorspitze.

Die schlanke Applikatorspitze ist vorzugsweise rotationssymmetrisch ausgebildet, wobei dies die Grundform meint. Auch eine Gestaltung, bei der an der Außenseite ein Gewinde oder anderweitige nachrangige Halterungsstrukturen zur Befestigung einer Kappe vorgesehen sind, die jedoch im Übrigen eine rotationssymmetrische Form aufweisen, sind als rotationssymmetrisch im Sinne der Erfindung zu begreifen. Die schlanke Form äußert sich darin, dass ihre Länge von einem Wurzelbereich bis zum distalen Ende vorzugsweise 2fach, insbesondere mindestens 2,5fach so groß ist wie der Außendurchmesser im Wurzelbereich. Der Wurzelbereich wird dabei durch jene Stelle gebildet, die beim Einführen in das Nasenloch nicht mehr eingeführt wird. Die Länge der Applikatorspitze vom Wurzelbereich bis zum distalen Ende beträgt vorzugsweise mindestens 15 mm. Es ist also insbesondere bevorzugt, dass die Applikatorspitze im Abstand von 15 mm vom distalen Ende einen Durchmesser von maximal 7,5 mm aufweist, vorzugsweise von maximal 6 mm.

Das Innenbauteil ist in das Außenbauteil eingesetzt. Es begrenzt gemeinsam mit dem Außenbauteil einen Flüssigkeitspfad, der zur Austragöffnung führt, insbesondere eine in diesem Flüssigkeitspfad vorgesehene Wirbelkammer stromaufwärts der Austragöffnung. Vorzugsweise wird die Wirbelkammer zumindest teilweise und insbesondere überwiegend durch eine Vertiefung im Innenbauteil gebildet. Das Innenbauteil kann zusätzlich einen Austragkanal bilden, der die Wirbelkammer mit einer Pumpeinrichtung verbindet. Bei anderen Gestaltungen ist der Austragkanal zwischen dem Innenbauteil und dem Außenbauteil angeordnet.

In die Wirbelkammer mündet mindestens ein drallgebender Einlaufkanal, wobei vorzugsweise mindestens zwei solche Einlaufkanäle vorgesehen sind, die einander gegenüberliegen, oder mehr als zwei Einlaufkanäle, die umfänglich verteilt in die vorzugsweise rotationssymmetrische Wirbelkammer münden. Die Einlaufkanäle sind gegenüber einer Radialrichtung angewinkelt, um die Flüssigkeit derart in die Wirbelkammer einströmen zu lassen, dass diese dort in eine rotierende Wirbelbewegung gelangt.

Der Einlaufkanal bzw. die Einlaufkanäle sind vorzugsweise durch Vertiefungen im Innenbauteil oder durch Durchbrechungen an einem nach innen weisenden Steg des Außenbauteils gebildet. Im Falle von den Einlaufkanälen, die durch Vertiefungen im Innenbauteil gebildet sind, ist es bevorzugt, wenn diese Vertiefungen eine geringere Tiefe als die sich anschließende ebenfalls durch eine Vertiefung gebildete Wirbelkammer aufweisen, um beim Einströmen der Flüssigkeit in die Wirbelkammer durch die aufgrund unterschiedlicher Tiefe bestehende Stufe einen Strömungsabriss zu verursachen.

Zur Erzielung eines Sprühstrahls, der von Kindern nicht als unangenehm empfunden wird, hat es sich als vorteilhaft herausgestellt, den Flüssigkeitsstrom zu reduzieren, also die Flüssigkeitsmenge je Zeiteinheit zu reduzieren, die durch die Austragöffnung hindurch ausgegeben wird. Vorzugsweise ist der Nasalspender derart ausgestaltet, dass bei einer üblichen Betätigung ein Flüssigkeitsstrom von weniger als 0,7 ml/Sekunde erzielt wird, vorzugsweise von weniger als 0,6 ml/Sekunde. Bei üblichen Nasalspendern für Erwachsene ist hingegen ein Flüssigkeitsstrom von 1,0 ml/Sekunde oder sogar 1,5 ml/Sekunden üblich. Trotz des verringerten Flüssigkeitsstroms müssen die Komponenten des Nasalspenders derart ausgelegt sein, dass die gewünschte Zerstäubung stattfindet.

Eine reine Reduktion des Flüssigkeitsstroms, beispielsweise durch eine Drossel im Austragkanal stromaufwärts der Wirbelkammer und der Einlaufkanäle, hat sich als nicht ideal herausgestellt, da durch den reduzierten Druck stromabwärts der Drossel die Bildung einer feinen Zerstäubung durch die Wirbelkammer erschwert wird.

Es werden zwei Alternativen zur Erzielung eines kindgerecht verringerten Flüssigkeitsstroms bei gleichzeitig guter Zerstäubung vorgeschlagen.

Eine erste Alternative betrifft einen Nasalapplikator, dessen Innenbauteil und Außenbauteil zueinander ortsfest angeordnet sind, beispielsweise indem sie durch eine Schnappverbindung fest miteinander verrastet sind. Sie definieren gemeinsam die Wirbelkammer und begrenzen gemeinsam die Einlaufkanäle in die Wirbelkammer umfänglich.

Bei einer solchen Konfiguration wird erfindungsgemäß vorgeschlagen, die Einlaufkanäle bzw. den mindestens einen Einlaufkanal besonders eng auszugestalten. Die im Einlaufkanal quer zur Strömungsrichtung ausgerichtete Querschnittsfläche des Einlaufkanals an dessen engster Stelle bzw. im Falle einer Mehrzahl von Einlaufkanälen die Summe der Querschnittsflächen der Einlaufkanäle an ihren jeweils engsten Stellen beträgt erfindungsgemäß nur 0,05 mm², vorzugsweise noch weniger, nämlich 0,04 mm² oder weniger.

Es wurde festgestellt, dass bereits eine solche Verengung zu einer geeigneten Verringerung des ausgetragenen Flüssigkeitsstroms führt sowie zu einer Beschleunigung der Flüssigkeit, durch die eine starke Zerstäubung bewirkt wird. Die sehr feinen Einlaufkanäle gestatten somit die zuverlässige Zerstäubung bei verringertem Flüssigkeitsstrom. Zudem sind die Einlaufkanäle als Ort der engsten Stelle eines Austragskanals besonders geeignet, da sie durch geeignete Formgebung des Innenbauteils und des Außenbauteils recht einfach und präzise mit dem gewünschten Querschnitt versehen werden können.

Der mindestens eine Einlaufkanal weist vorzugsweise einen sich in Strömungsrichtung verjüngenden Querschnitt auf, so dass die engste Stelle mit einem lichten Querschnitt von erfindungsgemäß maximal 0,05 mm² an einer Einmündung in die Wirbelkammer vorgesehen ist. Die Geschwindigkeit der Flüssigkeit erhöht sich demnach stetig, wenn sie in Strömungsrichtung in Richtung der Wirbelkammer strömt.

Auf die Größe der Austragsöffnung kommt es bei der beschriebenen Gestaltung mit sehr feinen Einlaufkanälen nicht maßgeblich an, da für die gute Zerstäubung in diesem Falle vor allem die Einlaufgeschwindigkeit der Flüssigkeit in die Wirbelkammer verantwortlich ist. Es wird daher vorgeschlagen, die Austragöffnung vergleichsweise groß zu gestalten. Die Austragöffnung sollte im Sinne einer einfacheren Herstellung an ihrer engsten Stelle eine Querschnittsfläche aufweisen, die größer als die Querschnittsfläche des Einlaufkanals bzw. als die Summe der Querschnittsflächen der Einlaufkanäle ist. Insbesondere vorzugsweise weist die Austragöffnung an ihrer engsten Stelle eine Querschnittsfläche von mindestens 0,06 mm² auf, insbesondere von mindestens 0,08 mm².

Die Einlaufkanäle sind bei dieser ersten Alternative umfänglich gemeinsam durch das Außenbauteil und das Innenbauteil gebildet. Sie werden gleichsam durch einen verbleibenden Spalt gebildet, der zwischen einer Stirnfläche des Innenbauteils und der Innenseite der Stirnwandung des Außenbauteils verbleibt. Insbesondere können in einer oberen Stirnfläche des Innenbauteils, die an der inneren Stirnwandung des Außenbauteil anliegt, Vertiefungen vorgesehen sein, die die Einlaufkanäle bilden. Die Einlaufkanäle werden durch einander gegenüberliegende Kanalwandungen beider Bauteile gebildet, des Außenbauteils und des Innenbauteils. Der Abstand dieser Kanalwandungen definiert die Kanalhöhe. Vorzugweise beträgt die Kanalhöhe weniger als 0,15 mm, vorzugsweise weniger als 0,12mm. Dieser enge Spalt lässt sich zuverlässig und bei geringem fertigungstechnischen Aufwand dadurch erzeugen, dass die genannten Vertiefungen entsprechender Tiefe an der Stirnseite des Innenbauteils vorgesehen sind.

Die Einlasskanäle sind vorzugsweise mit einer größeren Breite als Höhe versehen. Insbesondere vorzugsweise ist die Kanalbreite, also die Erstreckung der Querschnittsfläche des jeweiligen Einlaufkanals an dessen engster Stelle quer zur Strömungsrichtung und quer zur Kanalhöhe doppelt so groß wie die Kanalhöhe. Die Kanalbreite beträgt vorzugsweise mindestens 0,15 mm, insbesondere vorzugsweise 0,18 mm oder mehr.

Die zweite hier vorgeschlagene Alternative betrifft einen Nasalapplikator, bei dem das Innenbauteil beweglich im Außenbauteil angeordnet ist. Insbesondere ist das Innenbauteil linear verschiebbar im Außenbauteil angeordnet, wobei es insbesondere durch Erhöhung des Flüssigkeitsdrucks gegen die Kraft einer Rückstellfeder von der Austragöffnung weggedrückt wird.

Bei einer solchen Gestaltung mit verlagerbarem Innenbauteil ist es schwierig, die Einlaufkanäle derart auszulegen, dass sie eine reproduzierbare Drosselwirkung und hierdurch einen reduzierten Flüssigkeitsstrom erzeugen und zuverlässig zerstäuben, denn bei einer solchen Gestaltung begrenzen das Innenbauteil und das Außenbauteil gemeinsam den mindestens einen Einlaufkanal und der minimale Querschnitt des mindestens einen Einlaufkanals ist aufgrund der Beweglichkeit variabel.

Für einen solchen Fall mit verlagerbarem Innenbauteil wird erfindungsgemäß vorgeschlagen, die Austragöffnung in besonderer Form zu gestalten, um eine Drosselwirkung zu entfalten und gleichzeitig eine ausreichende Zerstäubung zu erzielen. Die Austragöffnung wird hierzu mit besonders geringem Querschnitt gestaltet, nämlich mit einer lichten Querschnittsfläche an ihrer engsten Stelle von maximal 0,05 mm², vorzugsweise von maximal 0,04 mm². Die Austragöffnung kann insbesondere eine rotationssymmetrische Form aufweisen.

Es ist demnach vorgesehen, dass die Zerstäubung nicht primär durch Beeinflussung der Geschwindigkeit beim Einströmen der Flüssigkeit in die Wirbelkammer beeinflusst wird, sondern durch eine besonders kleine Austragöffnung. Es hat sich gezeigt, dass auch dies die Zerstäubung und die Erzeugung eines konischen Sprühstrahls bei geringem Flüssigkeitsstrom ermöglicht. Insbesondere kann dies erreicht werden, wenn ein mittlerer Durchmesser der Austragöffnung zu einem maximalen Durchmesser der Pumpkammer in einem Verhältnis zwischen 1:3 und 1:5 steht.

Die Einlaufkanäle können insbesondere in einem die Austragöffnung innenseitig umgebenden Steg vorgesehen sein. In Abhängigkeit der Relativstellung des Innenbauteils kann dieses die Einlaufkanäle in Art einer innenseitigen Blende teilweise verschließen und somit den genannten variablen Querschnitt verursachen.

Das Innenbauteil und das Außenbauteil bilden vorzugsweise gemeinsam ein Austragventil aus. Das Innenbauteil weist dabei vorzugsweise einen Schließkolben auf, der in der Endlage den mindestens einen Einlaufkanal im Steg innenseitig überdeckt und dadurch ein Einströmen von Flüssigkeit aus dem Einlaufkanal in die Wirbelkammer in einer Endlage vollständig unterbindet. Alternativ oder zusätzlich kann vorgesehen sein, dass das Innenbauteil und das Außenbauteil in einer Endlage axial miteinander abdichten, indem eine Stirnfläche des Innenbauteils innenseitig an der Innenseite der Stirnwandung des Außenbauteils anliegt.

Die Herstellung einer erfindungsgemäß vorgeschlagenen kleinen Austragöffnung, die ein vergleichsweise großes Außenbauteil durchdringt, kann dadurch erleichtert werden, dass die Austragöffnung einen über die Dicke der Stirnwandung uneinheitlichen Querschnitt aufweist, also nicht über die gesamte Dicke der Stirnwandung den genannten geringen Querschnitt aufweist. Vorzugsweise weist die Austragöffnung an der Innenseite oder an der Außenseite der Stirnwandung, also eingangsseitig oder ausgangsseitig, eine insbesondere konische oder zylindrische Vertiefung auf, die gleichsam Teil der Austragöffnung ist oder eine Verjüngung der Stirnwandung darstellt. Diese Vertiefung erleichtert es, die gewünschte Präzision der Austragöffnung bei einem mittels Kunststoffspritzguss hergestellten Außenbauteil zu erzielen.

Ein erfindungsgemäßer Nasalspender trägt druckbeaufschlagte Flüssigkeit aus. Eine mögliche Bauweise sieht vor, dass die Flüssigkeit in einem als Druckspeicher ausgebildeten Flüssigkeitsspeicher gelagert ist und durch ein manuell betätigtes Ventil freigegeben wird. Bevorzugt ist es allerdings, dass die Flüssigkeit im Flüssigkeitsspeicher drucklos gespeichert ist und mittels einer Pumpeinrichtung zur Austragöffnung gefördert wird.

Insbesondere vorzugweise ist also eine Pumpeinrichtung vorgesehen, die mit einer Eingangsseite mit dem Flüssigkeitsspeicher verbunden ist und die über einen Austragskanal mit der Austragöffnung verbunden ist. Zur Betätigung der Pumpeinrichtung ist eine Betätigungshandhabe vorgesehen, die zwischen einer unbetätigten Endlage und einer betätigten Endlage verlagerbar ist, wobei diese Betätigungshandhabe vorzugsweise ortsfest zur Applikatorspitze vorgesehen ist und gemeinsam mit der Applikatorspitze gegenüber dem Flüssigkeitsspeicher niedergedrückt wird, um Flüssigkeit aus einer Pumpkammer der Pumpeinrichtung zur Austragöffnung zu fördern.

Die Pumpeinrichtung ist vorzugsweise eine Kolbenpumpe mit einer Zylinderwandung und einem hierzu abdichtenden Kolben, mittels dessen ein Pumpkammervolumen veränderbar ist. Eingangsseitig und ausgangseitig weist die Pumpeinrichtung ein Einlassventil und ein Auslassventil auf.

Das Auslassventil ist vorzugsweise als druckabhängig öffnendes Auslassventil ausgebildet, welches bei Überdruck in der Pumpkammer öffnet. Bei einer Gestaltung, bei der das Innenbauteil und das Außenbauteil gegeneinander beweglich sind und gemeinsam ein Austragventil bilden, kann dieses Austragventil gleichzeitig das Auslassventil der Pumpeinrichtung sein oder als zusätzliches Ventil zusätzlich zu einem separaten Auslassventil der Pumpeinrichtung vorgesehen sein.

Das Einlassventil kann ebenfalls ein druckabhängig öffnendes Ventil sein, welches bei Unterdruck in der Pumpkammer öffnet und hierdurch Flüssigkeit aus dem Flüssigkeitsspeicher ansaugt, insbesondere durch ein Steigrohr. Bevorzugt gegenüber einem druckabhängig öffnenden Einlassventil ist allerdings ein wegabhängig öffnendes Einlassventil. Dieses wird im Weiteren noch beschrieben.

Die Pumpeinrichtung ist vorzugsweise zur Förderung einer vergleichsweise geringen Flüssigkeitsmenge je Betätigung ausgelegt, um den Austrag für ein Kind nicht unangenehm erscheinen zu lassen. Bevorzugt ist es, dass der Nasalspender eher mehrfach nacheinander zur Applikation in das gleiche Nasenloch des Kindes verwendet wird, statt die gewünschte Flüssigkeitsmenge mit einer Betätigung vollständig auszutragen.

Insbesondere kann die Pumpeinrichtung dafür ausgebildet sein, bei einer Verlagerung der Betätigungshandhabe von der unbetätigten Endlage bis zur betätigten Endlage eine Flüssigkeitsmenge von weniger als 70 µl zur Austragöffnung zu fördern, insbesondere vorzugsweise von weniger als 50 µl oder sogar von weniger als 30 µl.

Dabei ist es insbesondere bevorzugt, wenngleich nicht zwingend, dass diese geringe Flüssigkeitsmenge im Zuge einer Betätigung aus der unbetätigten Endlage bis in die betätigte Endlage von mindestens 4 mm erfolgt, insbesondere von mindestens 6 mm erfolgt, wobei der eigentliche Fördervorgang nur während eines Teils dieser Betätigung stattfinden muss. Die vergleichsweise große Betätigungslänge erleichtert die Bedienung und senkt die erforderliche Betätigungskraft.

Wie eingangs schon erläutert, wird es bevorzugt, wenn ein der Nasalspender für einen vergleichsweise geringen Flüssigkeitsstrom ausgebildet ist, also insbesondere dafür, einen Austrag mit einem mittleren Flüssigkeitsstrom von weniger als 0,7 ml/Sekunde zu erzielen. Bei einer Gestaltung mit einer Pumpeinrichtung sind die Pumpeinrichtung und ein sich hier anschließender Austragkanal bis zur Austragöffnung daher vorzugsweise derart gestaltet, dass bei einer Betätigung mit einer Referenzkraft von 25 Newton die Betätigung aus der unbetätigten Endlage bis in die betätigte Endlage zu einem mittlerer Flüssigkeitsstrom von weniger als 0,7 ml/Sekunde führt, vorzugweise zu einem Flüssigkeitsstrom von weniger als 0,5 ml/Sekunde oder von weniger als 0,3 ml/Sekunde. Hierbei berücksichtigt ist nur jener Zeitraum, in dem ein Austrag stattfindet, also bei geöffnetem Auslassventil der Pumpeinrichtung.

Die Erfindung betrifft neben dem beschriebenen Nasalspender auch eine Pumpeinrichtung für einen Flüssigkeitsspender, insbesondere eine Pumpeinrichtung zur Verwendung in einem Nasalspender der oben beschriebenen Art. Besonders bevorzugt für einen Nasalspender der beschriebenen Art ist eine Pumpeinrichtung, die zur Ausbringung einer geringen und präzise dosierten Flüssigkeitsmenge geeignet ist.

Zur Erzielung dessen weist die erfindungsgemäße Pumpeinrichtung eine Pumpkammer auf, die durch zwei gegeneinander bewegliche Pumpenbauteil gebildet wird, insbesondere ein erstes Pumpenbauteil, welches Teil der Austrageinheit ist, und ein zweites Pumpenbauteil, welches Teil einer zum Flüssigkeitsspeicher ortsfesten Basis ist. Eine Verlagerung der Pumpenbauteile führt zu einer Veränderung des Volumens der Pumpkammer.

Die Pumpeinrichtung weist ein Einlassventil und ein Auslassventil auf, die die Pumpeinrichtung mit einem Flüssigkeitsspeicher bzw. einer Austragöffnung verbinden. Das Auslassventil ist vorzugsweise als druckabhängig öffnendes Auslassventil ausgebildet und kann identisch mit einem Austragventil des Spenders sein, welches durch das beschriebene Innenbauteil und das beschriebene Außenbauteil gebildet sein kann.

Das Einlassventil einer erfindungsgemäßen Pumpeinrichtung ist dagegen als wegabhängig öffnendes Einlassventil ausgebildet. Hierunter ist zu verstehen, dass in mindestens einer definierten Relativstellung der Pumpenbauteile das Einlassventil geöffnet und geschlossen wird. Das Einlassventil weist zu diesem Zweck einen Ventilkolben am ersten Pumpenbauteil und einen Dosierkanal am zweiten Pumpenbauteil auf. Bei einer Betätigung aus der Ausgangslage in die Endlage fährt der Ventilkolben zum Verschließen des Einlassventils und somit zum Bewirken des Austrags in den Dosierkanal abdichtend ein. Der Ventilkolben liegt also dichtend an der Innenseite des Dosierkanals an. Vorzugsweise sind der Ventilkolben und der Dosierkanal so bemessen, dass der Ventilkolben mit seiner Dichtfläche am gegenüberliegenden Ende wieder aus dem Dosierkanal ausrücken kann und somit eine schlagartige Beendigung des Austrags gegen Ende der Betätigung erzielt wird.

Wegabhängig öffnende Einlassventil sind grundsätzlich bereits bekannt, allerdings sind bekannte Ventile dieser Art für größere Flüssigkeitsströme ausgelegt. Um eine erfindungsgemäße Pumpeinrichtung der beschriebenen Art mit Dosierkanal und Ventilkolben mit einem geringen Fördervolumen zu gestalten, wird es als erfindungsgemäß bevorzugt angesehen, wenn die Pumpeinrichtung eine zylindrische Pumpkammerwandung am ersten Pumpenbauteil und eine innenseitig an der zylindrischen Pumpkammerwandung anliegenden Kolbenfläche am zweiten Pumpenbauteil aufweist, wobei dies baulich derart gelöst ist, das am zweiten Pumpenbauteil eine Hülse vorgesehen ist, die sowohl den Dosierkanal als auch die Kolbenfläche bildet. Die Hülse weist in einen Innenbereich der zylindrischen Pumpkammerwandung hinein. Die Innenseite der Hülse bildet den Dosierkanal. An der Außenseite der Hülse und/oder am distalen Ende der Hülse ist die Kolbenfläche vorgesehen, die im Betrieb dauerhaft an der zylindrischen Pumpkammerwandung anliegt.

Diese Bauform gestattet es, die Pumpkammer mit einem sehr geringen Querschnitt zu gestalten, da die beschriebene Hülse eine Doppelfunktion übernimmt. Dadurch, dass sie unmittelbar Träger der Kolbenfläche ist, kann die zylindrische Pumpkammerwandung mit einem nur geringfügig größeren Querschnitt als der Dosierkanal ausgestaltet sein. Entsprechend ist das Fördervolumen bezogen auf die Betätigungstrecke vergleichsweise gering.

Insbesondere kann vorgesehen sein, dass die Querschnittsfläche der zylindrischen Pumpkammerwandung zwischen 10 mm² und 20 mm² beträgt, vorzugsweise zwischen 10 mm² und 17 mm². Die Querschnittsfläche des Dosierkanals beträgt vorzugsweise zwischen 2 mm² und 5 mm², insbesondere zwischen 3 mm² und 4 mm². Die für die Flüssigkeitsförderung wirksame Kolbenfläche der Pumpkammer beträgt vorzugsweise zwischen 6 mm² und 15 mm². Im Falle einer wirksamen Kolbenfläche von exemplarisch 10 mm² wird eine Flüssigkeitsmenge von 50 µl über eine Dosierstrecke von 5 mm ausgetragen.

Die Erfindung betrifft einen Nasalspender der beschriebenen Art sowohl im unbefüllten Zustand vor Abfüllung als auch im für den Endkunden fertigen Zustand mit befülltem Flüssigkeitsspeicher. Der

Flüssigkeitsspeicher ist im befüllten Zustand vorzugsweise mit einer Gesamtflüssigkeitsmenge von weniger als 50 ml befüllt, vorzugsweise von weniger als 30 ml.

Im befüllten Zustand ist eine pharmazeutische Flüssigkeit in den Flüssigkeitsspender eingefüllt, wobei hierunter auch insbesondere salzhaltige wässrige Lösungen verstanden werden. Insbesondere kann die pharmazeutische Flüssigkeit auch eine Flüssigkeit mit abschwellender Wirkung sein, insbesondere eine Flüssigkeit mit einem Imidazolin-Bestandteil, wie beispielweise Oxymetazolin.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 und 2 zeigen ein erstes Ausführungsbeispiel eines erfindungsgemäßen Nasalspenders in ungeschnittener und geschnittener Darstellung.
Fig. 3 bis 5 zeigen die Austragöffnung sowie die der Austragöffnung vorgeschaltete Wirbelkammer des Nasalspenders der Fig. 1 und 2.
Fig. 6 und 7 zeigen ein zweites Ausführungsbeispiel eines erfindungsgemäßen Nasalspenders in ungeschnittener und geschnittener Darstellung.
Fig. 8 bis 10 zeigen die Austragöffnung sowie die der Austragöffnung vorgeschaltete Wirbelkammer des Nasalspenders der Fig. 6 und 7.

### DETAILLIERTE BESCHREIBUNG DER AUSFÜHRUNGSBEISPIELE

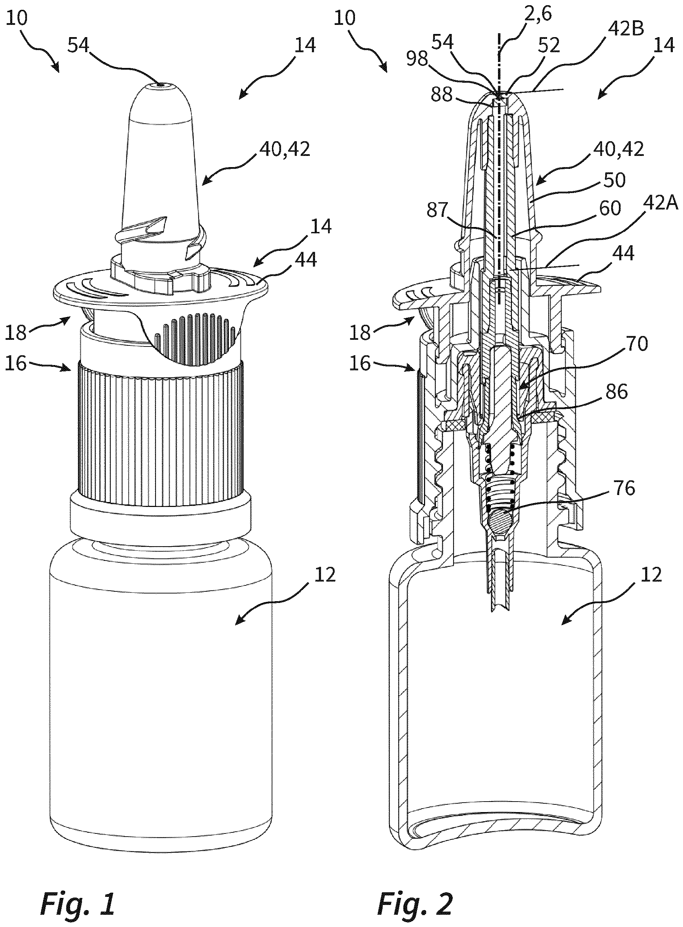

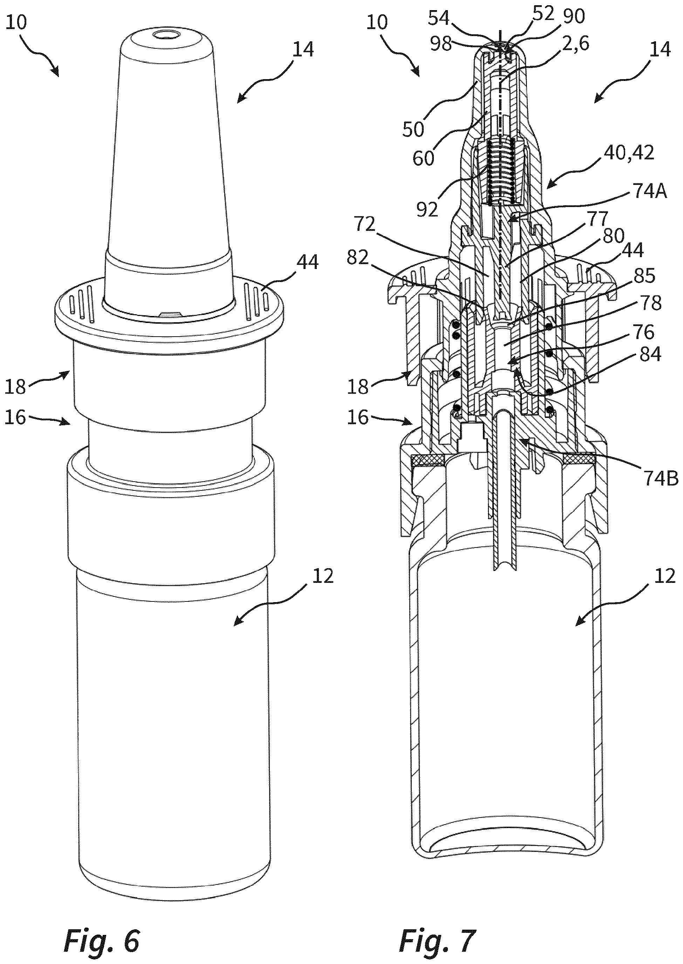

Fig. 1 bis 5 verdeutlichen ein erstes Ausführungsbeispiel eines erfindungsgemäßen Nasalspenders 10. Fig. 6 bis 10 verdeutlichen ein zweites Ausführungsbeispiel eines erfindungsgemäßen Nasalspenders 10.

Für beide Nasalspender 10 gilt, dass diese über einen Flüssigkeitsspeicher 12 verfügen, an dem ein Austragkopf 14 angebracht ist. Dieser Austragkopf 14 verfügt über eine Basis 16, die beispielsweise mittels einer Gewinde- oder einer Schnappverbindung mit dem Flüssigkeitsspeicher verbunden ist. Der Austragkopf 14 weist weiterhin eine Austrageinheit 18 auf, die beweglich an der Basis 16 angebracht ist und in Richtung der Basis 16 in einer Betätigungsrichtung 6 niedergedrückt werden kann. Die Austrageinheit 18 verfügt über einen schlanken Nasalapplikator 40 in Form einer sich zu einem distalen Ende hin verjüngenden Applikatorspitze 42, der dafür vorgesehen ist, in ein Nasenloch eines Benutzers eingeschoben zu werden. Weiterhin weist die Austrageinheit eine Betätigungshandhabe 44 in Form einer umlaufenden Fingerauflage auf.

Am distalen Ende der Applikatorspitze 42 ist eine Austragöffnung 54 vorgesehen, der eine Wirbelkammer 98 vorgeschaltet ist. Die Austragöffnung 54 ist als stirnseitige Durchbrechung eines Außenbauteils 50 ausgebildet. Die Wirbelkammer 98 wird durch die Innenseite einer Stirnwandung 52 dieses Außenbauteils sowie durch ein Innenbauteil 60 begrenzt.

Die Nasalspender verfügen jeweils über eine Pumpeinrichtung 70, die durch Niederdrücken der Austrageinheit 18 gegenüber dem Flüssigkeitsspeicher 12 und der Basis 16 betätigt werden kann. Die Pumpeinrichtung fördert Flüssigkeit aus dem Flüssigkeitsspeicher 12 zur Austragöffnung 54, die Flüssigkeit strömt durch Einlaufkanäle 96 in die Wirbelkammer 98 ein. Durch eine gegenüber einer Radialrichtung angewinkelte Ausrichtung der Einlaufkanäle 96 wird der Flüssigkeit bei Eintritt in die Wirbelkammer 98 ein Drall verliehen. Die in die Wirbelkammer 98 eingetretene Flüssigkeit rotiert daher mit hoher Geschwindigkeit im Uhrzeigersinn oder gegen den Uhrzeigersinn.

Die rotierende Flüssigkeit gelangt zur Stirnwandung 52 und der darin vorgesehenen Austragöffnung 54. Der Drall der Flüssigkeit bewirkt, dass die durch die Austragöffnung 54 austretende Flüssigkeit bei ausreichend hoher Geschwindigkeit in einen konischen Sprühstrahl aus feinen Einzeltröpfchen zerrissen wird.

Die in den Fig. 1 bis 5 bzw. 6 bis 10 dargestellten Nasalspender 10 stellen zwei bezüglich wesentlicher Details unterschiedliche Gestaltungen von Nasalspendern 10 dar, die insbesondere für die Verwendung bei Kindern vorgesehen sind. Bei den Nasalspendern 10 ist gemein, dass sie spezifisch ausgebildet sind für die Erzeugung eines ausgetragenen Flüssigkeitsstroms von nur etwa 0,7 ml/Sekunde. Dieser geringe Flüssigkeitsstrom sorgt zusammen mit einer feinen Zerstäubung dafür, dass der Austrag von Kindern nicht als sehr unangenehm empfunden wird. Je nachdem, wie weit der Flüssigkeitsstrom reduziert wird, kann sogar erreicht werden, dass der Austrag von Kindern kaum noch wahrgenommen wird.

Im Falle der Gestaltung der Fig. 1 bis 5 ist vorgesehen, dass die Austrageinheit 18 und der an ihr vorgesehene Nasalapplikator 40 durch im Wesentlichen zwei Bauteile gebildet wird, nämlich das Außenbauteil 50 und das Innenbauteil 60. Die Flüssigkeit wird durch eine Pumpeinrichtung 70 zur Austragöffnung 54 gefördert. Die Pumpeinrichtung 70 weist ein Einlassventil 76 und ein Auslassventil 86 auf. Jenseits des Auslassventil 86 strömt die Flüssigkeit in Richtung der Austragöffnung 54 durch einen Kanal 87, der in einem Teilabschnitt von dem Innenbauteil 60 umgeben ist. Am Ende dieses Kanals 87 tritt die Flüssigkeit durch radiale Öffnungen in einen endseitigen Kanalabschnitts 88 ein, der durch das Innenbauteil 60 und das Außenbauteil 50 gemeinsam begrenzt ist. Durch diesen Kanalabschnitt 88 gelangt die Flüssigkeit bis zu zwei Einlaufkanäle 96.

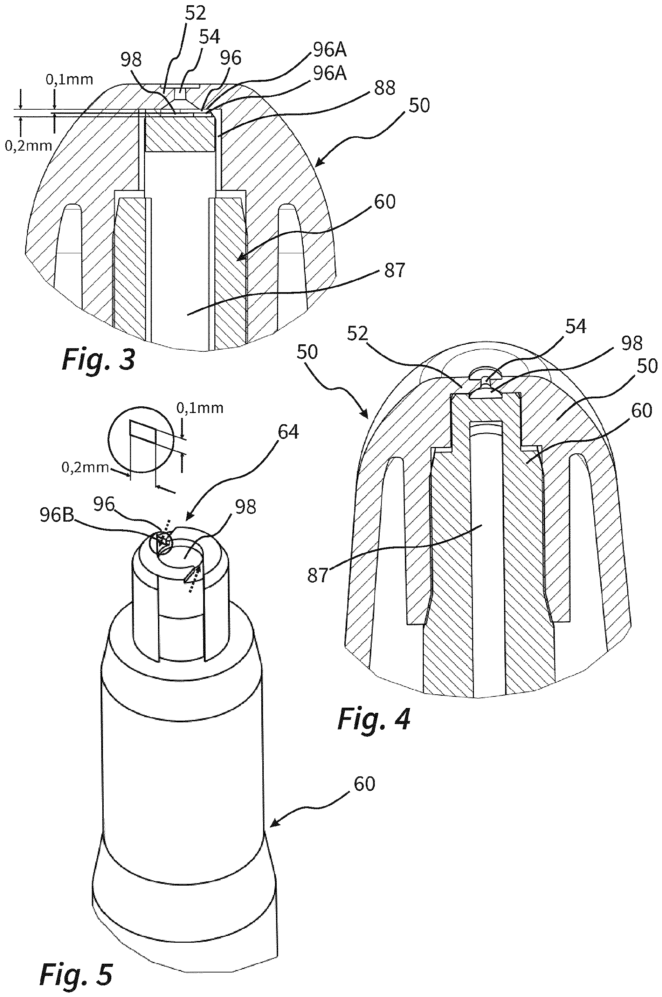

Die Einlaufkanäle 96 sind, wie insbesondere der Fig. 3 zu entnehmen ist, durch einander gegenüberliegende Kanalwandungen 96A, 96B begrenzt, wobei die in Fig. 3 gezeigte obere Kanalwandung 96A durch die Innenseite der Stirnwandung 52 des Außenbauteils 50 gebildet ist und wobei die in Fig. 3 gezeigte untere Kanalwandung 96B durch die Stirnfläche 64 des Innenbauteils 60 gebildet ist.

Die Stirnfläche 64 des Innenbauteils 60 ist insbesondere der Fig. 5 zu entnehmen. Die Stirnfläche ist mit einer zentrischen Vertiefung mit einer Tiefe von beispielsweise 0,2 mm ausgebildet, die zusammen mit der Stirnwandung 52 die Wirbelkammer 98 bildet. Die Einlaufkanäle 96 sind maßgeblich durch zwei weitere Vertiefungen gebildet, die eine geringere Tiefe von beispielsweise 0,1 mm aufweisen, so dass durch sie einströmende Flüssigkeit über eine Stufe in die Wirbelkammer 98 einströmt und dadurch ein sauberer Strömungsabriss verursacht wird.

Die Einlaufkanäle sind sich verjüngend bezogen auf eine Strömungsrichtung 4 geformt. Ihren engsten Querschnitt weisen sie unmittelbar vor der Einmündung in die Wirbelkammer 98 auf. Dort liegt eine Breite der Einlaufkanäle quer zur Strömungsrichtung 4 bei 0,2mm. Der Gesamtquerschnitt eines jeden der beiden Einlaufkanäle beträgt daher 0,02 mm², in Summe als 0,04 mm², wie sich aus dem in Fig. 5 dargestellten vergrößerten Bereich ersehen lässt

Die Austragöffnung 54 ist größer als die Summe der Querschnitte der Einlaufkanäle, so dass die Einlaufkanäle die insgesamt engste Stelle zwischen der Pumpeinrichtung 70 und dem Austritt der Flüssigkeit darstellen. Vorzugsweise ist die Austragöffnung rotationssymmetrische geformt und weist an ihrer engsten Stelle einen Durchmesser von mindestens 0,25 mm und vorzugsweise von mindestens 0,3 mm auf, also einen geringsten lichten Querschnitt von mindestens etwa 0,05 mm² und von vorzugsweise mindestens etwa 0,1 mm².

Die sehr engen Einlaufkanäle 96 verursachen in Kombination mit der Stufe am Übergang zwischen den Einlaufkanälen 96 und der Wirbelkammer 98 ein weitgehend störungsfreies Einströmen der Flüssigkeit mit hoher Geschwindigkeit. Gleichzeitig verringern die engen Einlaufkanäle 96 den Flüssigkeitsstrom, der bei üblicher Betätigungsgeschwindigkeit erzielt wird, vorzugsweise auf etwa 0,3 ml/Sekunde. Trotz dieses verringerten Flüssigkeitsstrom bewirkt die hohe Geschwindigkeit der Flüssigkeit beim Einströmen in die Wirbelkammer 98 eine sehr gute Zerstäubung. Auf den Durchmesser der Austragöffnung 54 kommt es daher kaum an. Im Falle der hier dargestellten Ausführungsform liegt der Durchmesser der Austragöffnung 54 bei etwa 0,3 mm. Es wären aber auch größere Durchmesser möglich, da die vorteilhafte Zerstäubung bei dieser Gestaltung vor allem von den Einlaufkanälen 96 gewährleistet wird.

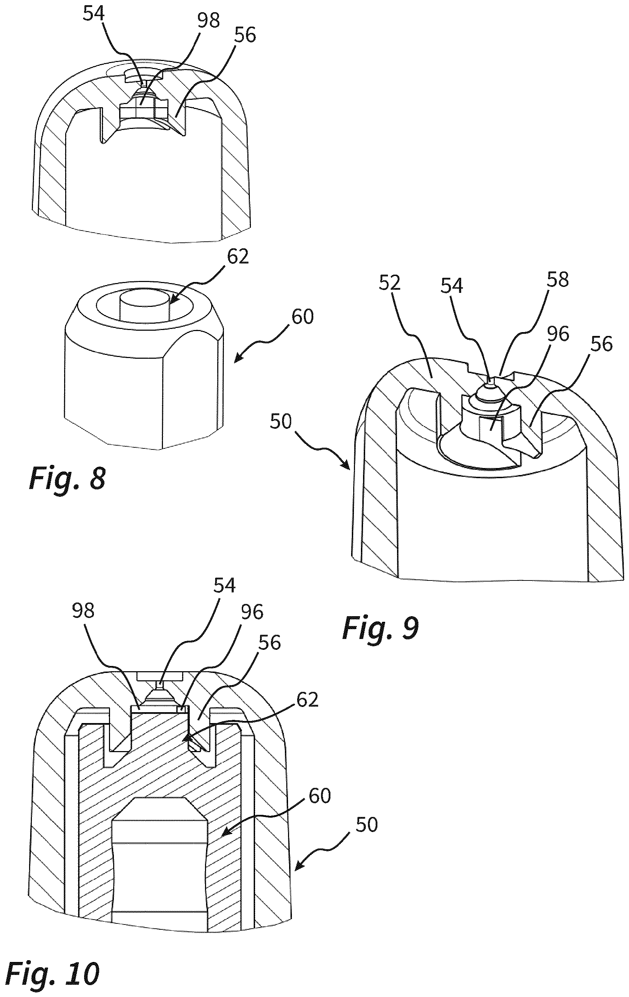

Im Falle der Gestaltung der Fig. 6 bis 10 ist vorgesehen, dass das Außenbauteil 50 und das Innenbauteil 60 gegeneinander beweglich sind und gemeinsam ein Austragventil 90 bilden. Wie anhand der Fig. 8 bis 10 und insbesondere anhand von Fig. 9 zu erkennen ist, ist innenseitig an der Stirnwandung 52 ein umlaufender Steg 56 vorgesehen, der durch Schlitze unterbrochen ist, die zusammen mit dem Innenbauteil 60 die Einlaufkanäle 96 bilden. Das Innenbauteil 60 bildet mit seinem distalen Ende einen Schließkolben 62, der gegenüber dem Steg 56 beweglich ist. Sofern kein Flüssigkeitsdruck anliegt, ist das Austragventil 90 geschlossen. Der Schließkolben ist in diesem Zustand derart weit in den Steg 56 eingerückt, dass die dortigen Schlitze vollständig überdeckt sind und somit keine Flüssigkeit in die vom Steg und dem Schließkolben begrenzte Wirbelkammer 98 einströmen kann. Wird mittels der Pumpeinrichtung 70 des Nasalspenders 10 Flüssigkeit druckbeaufschlagt, so drückt der Flüssigkeitsdruck das Innenbauteil 60 gegen die Kraft einer Ventilfeder 92 von der Austragöffnung 54 weg, so dass die Wirbelkammer 98 vergrößert wird und der Zugang für die Flüssigkeit durch die Einlaufkanäle 96 geöffnet wird.

Da aufgrund der Druckabhängigkeit der Öffnung der Einlaufkanäle diese als Verengung zur Erzielung einer reproduzierbaren Drosselwirkung nicht gut geeignet sind, ist bei der Gestaltung der Fig. 6 bis 10 dagegen die Austragöffnung 54 sehr klein gestaltet. Es hat sich gezeigt, dass sich auch hierdurch die Erzielung eines geringen Flüssigkeitsstroms und die wunschgemäße Zerstäubung zu erzielen sind.

Die Austragöffnung 54 weist daher an ihrer engsten Stelle eine Querschnittsfläche von 0,04 mm² oder weniger auf. Insbesondere vorzugsweise ist die Austragöffnung rotationssymmetrisch und mit einem geringsten Durchmesser von 0,2 mm und entsprechend mit einer Querschnittsfläche von weniger als 0,035 mm² ausgebildet.

Zur Erleichterung der Herstellung einer an der engsten Stelle derart feinen Austragöffnung ist vorgesehen, dass dies Stirnwandung 52 im Bereich der Austragöffnung auf der Innenseite und/oder der Außenseite Vertiefungen 58 aufweist. Die maximale Dicke der Stirnwandung von ca. 0,5 mm bis 0,8 mm ist also im Bereich der Austragöffnung durch die Vertiefungen 58 vermindert, vorzugsweise auf eine Dicke zwischen 0,2 mm und 0,4 mm. Diese Dicke von vorzugsweise zwischen 0,2 mm und 0,4 mm ist weiterhin vorzugsweise durch einen zylindrischen Abschnitt der Austragöffnung 54 durchdrungen, der durchgängig die genannte geringe Querschnittsfläche von weniger als 0,04 mm² aufweist, vorzugsweise von weniger als 0,035 mm².

Die Wirbelkammer 98 weist vorzugsweise einen maximalen Durchmesser auf, der etwa viermal bis achtmal so groß ist wie der Durchmesser der Austragöffnung 54 und daher insbesondere vorzugsweise zwischen 0,8 mm und 2,0 mm beträgt, insbesondere zwischen 0,8 mm und 1,2 mm. Der Wirbelkammer 98 geht vorzugsweise über einen konisch verengten Bereich, insbesondere gebildet durch die genannte innenseitige Vertiefung, in der genannten zylindrischen Abschnitt der Austragöffnung 54 über.

Bei Betätigung des Nasalspenders 10 durch Hinabdrücken der Austrageinheit 18 öffnen sich in der in Fig. 10 verdeutlichten Weise druckbedingt die Einlaufkanäle 96, da der Schließkolben 62 von der Austragöffnung beabstandet wird. Die Flüssigkeit strömt in die Wirbelkammer 98 ein und wird durch die sehr enge Austragöffnung 54 ausgetragen. Obwohl die Einlaufkanäle je nach Stellung des Schließkolbens deutlich größer als bei der Gestaltung der Fig. 1 bis 5 sind, wird aufgrund der engen Austragöffnung 54 eine erheblicher Betätigungswiderstand sowie die gewünschte Zerstäubung bei gleichzeitig geringem Flüssigkeitsstrom von weniger als 0,3 ml/Sekunde erreicht.

Neben der Gestaltung des Nasalspenders 10 der Fig. 6 bis 10 in Hinblick auf die Wirbelkammer 8 und die Austragöffnung 54 ist die Pumpeinrichtung 70 in besonderer Weise gestaltet. Die Pumpeinrichtung weist ein Einlassventil 76 und ein Auslassventil 90 auf. Das Auslassventil 90 ist mit dem bereits genannten Austragventil 90 identisch. Es sind jedoch auch Gestaltungen mit zwei unabhängigen Ventilen stromabwärts der Pumpkammer 72 der Pumpeinrichtung 70 möglich.

Das Einlassventil 76 ist abweichend von der Gestaltung der Fig. 1 bis5 kein druckabhängig öffnendes Einlassventil, sondern ein wegabhängig öffnendes.

Es umfasst ein erstes Pumpenbauteil 74A, welches Teil der verlagerbaren Austrageinheit 18 ist und unter Einschluss des Innenbauteils 60 fest mit dem Außenbauteil 50 verbunden ist. Dieses erste Pumpenbauteil 74A verfügt über einen zentrischen Ventilkolben 77 und einen diesen Ventilkolben 77 umgebenden umlaufenden Steg, dessen Innenseite eine Pumpkammerwandung 80 bildet.

Ein zweites Pumpenbauteil 74B ist Teil der Basis 16. Dieses zweite Pumpenbauteil 74B verfügt über eine zentrische Hülse 84, die folgende Funktionen übernimmt: Die Hülse 84 ist von einem Einlasskanal 85 durchdrungen, wobei ein Abschnitt des Einlasskanals 85 mit geringem Querschnitt einen Dosierkanal 78 bildet. Dieser Dosierkanal ist so ausgelegt, dass der Ventilkolben 77 des ersten Pumpkammerbauteils bei Anordnung im Dosierkanal hierdurch den Einlasskanal 85 verschließt und die kommunizierende Verbindung zwischen der Pumpkammer 72 und dem Flüssigkeitsspeicher 12 unterbricht.

Die Hülse 84 ist darüber hinaus Träger einer Kolbenfläche 82, die am distalen Ende der Hülse vorgesehen ist und den maximalen Hülsendurchmesser definiert. Die Kolbenfläche 82 liegt an der Pumpkammerwandung 80 an.

Wird nun die Austrageinheit 18 niedergedrückt, so wird zunächst die Flüssigkeit aus der Pumpkammer 72 durch den noch offenen Einlasskanal 85 zurück in den Flüssigkeitsspeicher 20 gedrückt, bis der Ventilkolben 77 in den Dosierkanal 78 eintritt. Der Rückfluss ist nun unterbrochen und das fortgesetzte Niederdrücken der Austrageinheit 18 bewirkt eine Druckerhöhung in der Pumpkammer 72 und entsprechend ein Öffnen des Auslass- und Austragventils 90. Dieser Austrag findet über eine Betätigungsstrecke von 5 mm statt, was durch die Länge des Dosierkanals 78 bedingt ist. Sobald der Ventilkolben 77 am unteren Ende aus dem Dosierkanal 78 austritt, ist die kommunizierende Verbindung zwischen der Pumpkammer 72 und dem Flüssigkeitsspeicher 12 wieder hergestellt. Der Überdruck in der Pumpkammer entlädt sich in den Flüssigkeitsspeicher 12 und das Austrag- und Auslassventil 90 schließt.

Während des Austrags, also während des Zeitraums zwischen dem Schließen des Einlassventils 76 und dem Öffnen des Einlassventils 76, wird die Flüssigkeit aus der Pumpkammer 72 in Richtung der Austragöffnung 54 gefördert.

Dadurch, dass die Hülse 84 eine Doppelfunktion übernimmt und selbst die Kolbenfläche 82 trägt, ist eine Pumpkammer mit sehr geringem Durchmesser erzielbar. Vorliegend liegt der Durchmesser der zylindrischen Pumpkammerwandung bei 4 mm und die Querschnittsfläche somit bei etwa 12,5 mm². Die wirksame Kolbenfläche unter Berücksichtigung des Ventilkolbens 77 ist noch etwas geringer und liegt bei etwa 7 mm² bis 10 mm², vorliegend bei 8 mm². Über die Dosierstrecke von 5 mm werden dementsprechend 40 µl Flüssigkeit gefördert und ausgetragen.

Durch die Geometrie des Flüssigkeitspfades von der Pumpkammer 72 bis zur Austragöffnung 54 wird erreicht, dass der Austrag bei einer Referenz-Betätigungskraft von 25 Newton über einen Zeitraum von ca. 0,15 Sekunden erfolgt, also mit einem mittleren Flüssigkeitsstrom von 0,27 ml/Sekunde. Dies führt zu einem weichen und in der Nase fast unspürbaren Sprühstrahl, der von Kindern nicht als sehr unangenehm empfunden wird.

## Patentansprüche

1. Nasalspender (10) zur nasalen Applikation von Flüssigkeiten in zerstäubter Form, insbesondere zur nasalen Applikation von Flüssigkeiten bei Kindern, mit den folgenden Merkmalen:
a. der Nasalspender (10) verfügt über einen Flüssigkeitsspeicher (12) zur Aufnahme von Flüssigkeit vor der Abgabe, und
b. der Nasalspender (10) verfügt über einen Nasalapplikator (40) zur Abgabe der Flüssigkeit, und
c. ein Außenbauteil (50) des Nasalapplikators (40) weist eine schlanke Applikatorspitze (42) mit einer distalen Stirnwandung (52) auf, die von einer Austragöffnung (54) durchdrungen ist, welche zur Abgabe eines Sprühstrahls in einer Austragrichtung (2) ausgebildet ist, und
d. ein Innenbauteil (60) des Nasalapplikators (40) ist in das Außenbauteil (50) eingesetzt, und
e. das Außenbauteil (50) und das Innenbauteil (60) begrenzen gemeinsam eine Wirbelkammer (98) stromaufwärts der Austragöffnung (54), wobei mindestens ein drallgebender Einlaufkanal (96) vorgesehen ist, der in die Wirbelkammer (98) mündet,
**gekennzeichnet durch** eines der folgenden weiteren Merkmale:
f. das Innenbauteil (60) und das Außenbauteil (50) sind zueinander ortsfest angeordnet und begrenzen gemeinsam den mindestens einen Einlaufkanal (96) umfänglich, wobei die quer zur Strömungsrichtung (4) im Einlaufkanal ausgerichtete Querschnittsfläche des Einlaufkanals (96) an seiner engsten Stelle bzw. die Summe der quer zur Strömungsrichtung (4) in den Einlaufkanälen (96) ausgerichtete Querschnittsflächen der Einlaufkanäle an ihren jeweils engsten Stellen maximal 0,05 mm² beträgt, vorzugsweise von maximal 0,04 mm², oder
g. das Innenbauteil (60) ist beweglich im Außenbauteil (50) angeordnet und das Innenbauteil (60) und das Außenbauteil (50) begrenzen gemeinsam den mindestens einen Einlaufkanal (96), so dass der Querschnitt des mindestens einen Einlaufkanals (96) durch die Beweglichkeit des Innenbauteils (60) gegenüber dem Außenbauteil (50) variabel ist, und die Austragöffnung (54) weist an ihrer engsten Stelle eine Querschnittsfläche von maximal 0,05 mm² auf, vorzugsweise von maximal 0,04 mm².

2. Nasalspender (10) nach Anspruch 1 mit den folgenden weiteren Merkmalen:
a. das Innenbauteil (60) und das Außenbauteil (50) sind zueinander ortsfest angeordnet, und
b. die Austragöffnung (54) weist an ihrer engsten Stelle eine Querschnittsfläche auf, die größer als die Querschnittsfläche des Einlaufkanals (96) bzw. als die Summe der Querschnittsflächen der Einlaufkanäle (96) ist.

3. Nasalspender (10) nach Anspruch 2 mit den folgenden weiteren Merkmalen:
a. die Austragöffnung (54) weist an ihrer engsten Stelle eine Querschnittsfläche von mindestens 0,06 mm² auf.
vorzugsweise mit dem folgenden zusätzlichen Merkmal:
b. die Austragöffnung (54) weist an ihrer engsten Stelle eine Querschnittsfläche von mindestens 0,08 mm² auf.

4. Nasalspender (10) nach einem der Ansprüche 1 bis 3 mit den folgenden weiteren Merkmalen:
a. das Innenbauteil (60) und das Außenbauteil (50) sind zueinander ortsfest angeordnet, und
b. der mindestens eine Einlaufkanal (96) ist an seiner engsten Stelle derart gestaltet, dass zwei einander gegenüberliegende Kanalwandungen (96A, 96B) des Einlaufkanals (96), von denen eine durch das Innenbauteil (60) und eine durch das Außenbauteil (50) gebildet wird, eine Kanalhöhe von weniger als 0,15 mm definieren, vorzugsweise von weniger als 0,12mm,
vorzugsweise mit dem folgenden zusätzlichen Merkmal:
c. quer zur Kanalhöhe weist der mindestens eine Einlaufkanal (96) am engsten Querschnitt eine Kanalbreite auf, die größer als die Kanalhöhe ist und vorzugsweise mindestens doppelt so breit wie die Kanalhöhe ist, wobei die Kanalbreite vorzugsweise mindestens 0,15 mm beträgt, insbesondere vorzugsweise 0,18 mm oder mehr.

5. Nasalspender (10) nach einem der Ansprüche 1 bis 4 mit den folgenden weiteren Merkmalen:
a. das Innenbauteil (60) und das Außenbauteil (50) sind zueinander ortsfest angeordnet, und
b. die Wirbelkammer (98) wird durch eine Vertiefung im Innenbauteil (60) gebildet, wobei der mindestens eine Einlaufkanal (96) durch eine weitere Vertiefung im Innenbauteil (60) mit geringerer Tiefe gebildet ist.

6. Nasalspender (10) nach Anspruch 1 mit den folgenden weiteren Merkmalen:
a. das Innenbauteil (60) ist beweglich im Außenbauteil (50) angeordnet, und
b. das Innenbauteil (60) und das Außenbauteil (50) sind bis in eine Endlage verlagerbar, in der ein Zufluss in die Wirbelkammer (98) unterbunden ist,
vorzugsweise mit dem folgenden zusätzlichen Merkmal:
c. das Innenbauteil (60) und das Außenbauteil (50) bilden gemeinsam ein mittels Flüssigkeitsdruck schaltbares Austragventil.

7. Nasalspender (10) nach Anspruch 1 oder 6 mit den folgenden weiteren Merkmalen:
a. das Innenbauteil (60) ist beweglich im Außenbauteil (50) angeordnet, und
b. das Außenbauteil (50) weist an der Innenseite der Stirnwandung (52) einen die Austragöffnung (54) umgebenden Steg (56) auf, der von mindestens einem Einlaufkanal (96) durchdrungen ist, und
c. das Innenbauteil (60) weist einen Schließkolben (62) auf, der in der Endlage den mindestens einen Einlaufkanal (96) innenseitig überdeckt und dadurch ein Einströmen von Flüssigkeit aus dem Einlaufkanal (96) in die Wirbelkammer (98) unterbindet oder in der Endlage mit einer Stirnfläche (64) innenseitig an der Innenseite der Stirnwandung (52) des Außenbauteils (50) anliegt.

8. Nasalspender (10) nach Anspruch 1, 6 oder 7 mit den folgenden weiteren Merkmalen:
a. das Innenbauteil (60) ist beweglich im Außenbauteil (50) angeordnet, und
b. die Austragöffnung (54) weist einen sich in Austragrichtung (2) von einer Innenseite der Stirnwandung (52) zu einer Außenseite der Stirnwandung (52) veränderlichen Querschnitt auf, wobei an der Innenseite und/oder der Außenseite eine Vertiefung mit gegenüber der engsten Stelle der Austragöffnung (54) größerer Querschnittsfläche gebildet ist, insbesondere eine zylindrische und/oder eine konische Vertiefung (58).

9. Nasalspender (10) nach einem der vorstehenden Ansprüche mit dem folgenden weiteren Merkmal:
a. die Applikatorspitze (42) ist rotationssymmetrisch und sich zum distalen Ende verjüngend ausgebildet, wobei ihre Länge von einem Wurzelbereich bis zum distalen Ende mindestens doppelt so groß ist wie der Außendurchmesser im Wurzelbereich,
vorzugsweise mit dem folgenden zusätzlichen Merkmal:
b. die Länge der Applikatorspitze (42) vom Wurzelbereich (42A) bis zum distalen Ende (42B) beträgt mindestens 15 mm.

10. Nasalspender (10) nach einem der vorstehenden Ansprüche mit den folgenden weiteren Merkmalen:
a. der Nasalspender (10) verfügt über eine Pumpeinrichtung (70), die mit einer Eingangsseite mit dem Flüssigkeitsspeicher (12) verbunden ist und die über einen Austragskanal mit der Austragöffnung (54) verbunden ist, und
b. zur Betätigung der Pumpeinrichtung (70) ist eine Betätigungshandhabe (44) vorgesehen, die zwischen einer unbetätigten Endlage und einer betätigten Endlage verlagerbar ist, und
c. die Pumpeinrichtung (70) ist derart ausgelegt, dass bei einer Verlagerung der Betätigungshandhabe (44) von der unbetätigten Endlage bis zur betätigten Endlage eine Flüssigkeitsmenge von weniger als 70 µl zur mindestens einen Austragöffnung (54) gefördert wird.
vorzugsweise mit mindestens einem der folgenden Merkmale:
d. die Pumpeinrichtung (70) ist derart ausgelegt, dass bei einer Verlagerung der Betätigungshandhabe (44) von der unbetätigten Endlage bis zur betätigten Endlage eine Flüssigkeitsmenge von weniger als 50 µl zur mindestens einen Austragöffnung (54) gefördert wird, insbesondere weniger als 30 µl, und/oder
e. die unbetätigte Endlage und die betätigte Endlage sind um mindestens 4 mm voneinander beabstandet, vorzugsweise um mindestens 6 mm, und/oder
f. die Pumpeinrichtung (70) ist als Kolbenpumpe miteinem Pumpenzylinder und einem darin zur Verringerung eines Pumpkammervolumens verlagerbaren Pumpkolben ausgebildet und/oder
g. die Pumpeinrichtung (70) ist gemäß einem der Ansprüche 14 oder 15 ausgebildet.

11. Nasalspender (10) nach Anspruch 10 mit dem folgenden weiteren Merkmal:
a. die Pumpeinrichtung (70) und der Austragskanal zur Austragöffnung (54) sind derart ausgelegt, dass bei einer Betätigungskraft von 25 Newton die Betätigung aus der unbetätigten Endlage bis in die betätigte Endlage zu einem mittlerer Flüssigkeitsstrom von weniger als 0,7 ml/Sekunde führt.

12. Nasalspender (10) nach einem der vorstehenden Ansprüche mit dem folgenden weiteren Merkmal:
a. der Nasalapplikator (40) ist Teil einer gegenüber dem Flüssigkeitsspeicher (12) linear in einer Betätigungsrichtung (6) parallel zur Austragrichtung (2) verlagerbaren Austrageinheit (18) auf.

13. Nasalspender (10) nach einem der vorstehenden Ansprüche mit mindestens einem der folgenden weiteren Merkmale:
a. es ist eine Mehrzahl von Einlaufkanälen (96) vorgesehen, vorzugsweise zwei einander gegenüberliegende Einlaufkanäle (96), und/oder
b. die Wirbelkammer (98) weist eine rotationssymmetrische Innenkontur auf, und/oder
c. die Wirbelkammer (98) wird durch eine Vertiefung im Innenbauteil (60) gebildet, wobei der mindestens eine Einlaufkanal (96) vorzugweise durch eine weitere Vertiefung im Innenbauteil (60) mit geringerer Tiefe gebildet ist, und/oder
d. der mindestens eine Einlaufkanal (96) weist einen sich in Strömungsrichtung (4) verjüngenden Querschnitt auf, so dass die engste Stelle an einer Einmündung in die Wirbelkammer (98) vorgesehen ist.

14. Pumpeinrichtung (70) für einen Flüssigkeitsspender, insbesondere einen Flüssigkeitsspender nach einem der vorstehenden Ansprüche, mit den folgenden Merkmalen:
a. die Pumpeinrichtung (70) weist eine Pumpkammer (72) auf, die durch zwei gegeneinander bewegliche Pumpenbauteil (74A, 74B) gebildet wird, und
b. die Pumpeinrichtung (70) weist ein Einlassventil (76) und ein Auslassventil (90) auf, die die Pumpeinrichtung (70) mit einem Flüssigkeitsspeicher (12) und einer Austragöffnung (54) verbinden, und
c. das Auslassventil (90) ist als druckabhängig öffnendes Auslassventil (90) ausgebildet, und
d. das Einlassventil (76) weist einen Ventilkolben (77) am ersten Pumpenbauteil (74A) und einen Dosierkanal (78) am zweiten Pumpenbauteil (74B) auf, in welchen der Ventilkolben (77) zum Verschließen des Einlassventils (76) im Zuge einer Pumpbetätigung einfährt, **gekennzeichnet durch** die folgenden weiteren Merkmale:
e. die Pumpeinrichtung (70) weist einen zylindrische Pumpkammerwandung (80) am ersten Pumpenbauteil (74A) auf, und
f. die Pumpeinrichtung (70) weist eine innenseitig an der zylindrischen Pumpkammerwandung (80) anliegenden Kolbenfläche (82) auf, und
g. am zweiten Pumpenbauteil (74B) ist eine Hülse (84) vorgesehen, die in einen Innenbereich der zylindrische Pumpkammerwandung (80) hineinragt und deren Innenseite den Dosierkanal (78) bildet und an deren Außenseite und/oder an deren distalem Ende die Kolbenfläche (82) vorgesehen ist.

15. Pumpeinrichtung (70) nach Anspruch 14 mit dem folgenden weiteren Merkmal:
a. die Querschnittsfläche der zylindrischen Pumpkammerwandung (80) beträgt 10 mm² und 20 mm², vorzugsweise zwischen 10 mm² und 17 mm²,
vorzugsweise mit dem folgenden zusätzlichen Merkmal:
b. die Querschnittsfläche des Dosierkanals (78) beträgt zwischen 2 mm² und 5 mm², vorzugsweise zwischen 3 mm² und 4 mm².

## Claims

1. Nasal dispenser (10) for the nasal application of liquids in atomized form, in particular for the nasal application of liquids for children, comprising the following features:
a. the nasal dispenser (10) has a liquid reservoir (12) for receiving liquid be fore it is discharged, and
b. the nasal dispenser (10) has a nasal applicator (40) for discharging the liq uid, and
c. an outer component (50) of the nasal applicator (40) has a slender applica tor tip (42) with a distal end wall (52), through which passes an outlet open ing (54) designed to discharge a spray jet in a discharge direction (2), and
d. an inner component (60) of the nasal applicator (40) is inserted in the outer component (50), and
e. the outer component (50) and the inner component (60) together delimit a vortex chamber (98) upstream of the outlet opening (54), wherein at least one swirl-imparting inlet channel (96) emerging into the vortex chamber (98) is provided,
**characterized by** one of the following further features:
f. the inner component (60) and the outer component (50) are arranged in a fixed position in relation to one another and together circumferentially de limit the at least one inlet channel (96), wherein the cross-sectional area, aligned transversely to the flow direction (4) in the inlet channel, of the inlet channel (96) at its narrowest point, or the sum of the cross-sectional areas, aligned transversely to the flow direction (4) in the inlet channels (96), of the inlet channels at their respective narrowest points, is at most 0.05 mm², preferably at most 0.04 mm², or
g. the inner component (60) is arranged movably in the outer component (50) and the inner component (60) and the outer component (50) together de limit the at least one inlet channel (96), with the result that the cross section of the at least one inlet channel (96) can be varied by virtue of the movabil ity of the inner component (60) with respect to the outer component (50), and the outlet opening (54) at its narrowest point has a cross-sectional area of at most 0.05 mm ², preferably at most 0.04 mm ².

2. Nasal dispenser (10) according to Claim 1, comprising the following further features:
a. the inner component (60) and the outer component (50) are arranged in a fixed position in relation to one another, and
b. the outlet opening (54) at its narrowest point has a cross-sectional area which is greater than the cross-sectional area of the inlet channel (96), or than the sum of the cross-sectional areas of the inlet channels (96).

3. Nasal dispenser (10) according to Claim 2, comprising the following further features:
a. the outlet opening (54) at its narrowest point has a cross-sectional area of at least 0.06 mm ².
preferably comprising the following additional feature:
b. the outlet opening (54) at its narrowest point has a cross-sectional area of at least 0.08 mm ².

4. Nasal dispenser (10) according to one of Claims 1 to 3, comprising the following further features:
a. the inner component (60) and the outer component (50) are arranged in a fixed position in relation to one another, and
b. the at least one inlet channel (96) at its narrowest point is configured such that two opposite channel walls (96A, 96B) of the inlet channel (96), one of which is formed by the inner component (60) and one of which is formed by the outer component (50), define a channel height of less than 0.15 mm, preferably less than 0.12 mm,
preferably comprising the following additional feature:
c. transversely in relation to the channel height, the at least one inlet channel (96) at the narrowest cross section has a channel width which is greater than the channel height and preferably is at least twice as wide as the channel height, the channel width being preferably at least 0.15 mm, in par ticular preferably 0.18 mm or more.

5. Nasal dispenser (10) according to one of Claims 1 to 4, comprising the following further features:
a. the inner component (60) and the outer component (50) are arranged in a fixed position in relation to one another, and
b. the vortex chamber (98) is formed by a depression in the inner component (60), wherein the at least one inlet channel (96) is formed by a further de pression in the inner component (60) with a smaller depth.

6. Nasal dispenser (10) according to Claim 1, comprising the following further features:
a. the inner component (60) is arranged movably in the outer component (50), and
b. the inner component (60) and the outer component (50) can be displaced into an end position in which an inflow into the vortex chamber (98) is pre vented,
preferably comprising the following additional feature:
c. the inner component (60) and the outer component (50) together form an outlet valve that can be switched by means of liquid pressure.

7. Nasal dispenser (10) according to Claim 1 or 6, comprising the following further features:
a. the inner component (60) is arranged movably in the outer component (50), and
b. the outer component (50) has, on the inner side of the end wall (52), a flange (56) which surrounds the outlet opening (54) and through which at least one inlet channel (96) passes, and
c. the inner component (60) has a closing piston (62), which in the end posi tion covers the at least one inlet channel (96) on the inside and as a result prevents liquid from flowing from the inlet channel (96) into the vortex chamber (98) or in the end position bears against the inner side of the end wall (52) of the outer component (50) by way of an end face (64).

8. Nasal dispenser (10) according to Claim 1, 6 or 7, comprising the following further features:
a. the inner component (60) is arranged movably in the outer component (50), and
b. the outlet opening (54) has a cross section which varies in the discharge direction (2) from an inner side of the end wall (52) to an outer side of the end wall (52), wherein a depression with a cross-sectional area which is greater than the narrowest point of the outlet opening (54), in particular a cylindrical and/or a conical depression (58), is formed on the inner side and/or the outer side.

9. Nasal dispenser (10) according to one of the preceding claims, comprising the following further feature:
a. the applicator tip (42) has a rotationally symmetrical form and tapers to wards the distal end, its length from a root region to the distal end being at least twice the outside diameter in the root region,
preferably comprising the following additional feature:
b. the length of the applicator tip (42) from the root region (42A) to the distal end (42B) is at least 15 mm.

10. Nasal dispenser (10) according to one of the preceding claims, comprising the following further features:
a. the nasal dispenser (10) has a pump device (70), which is connected to the liquid reservoir (12) by way of an inlet side and is connected to the outlet opening (54) via an outlet channel, and
b. an actuator (44) which can be displaced between an unactuated end posi tion and an actuated end position is provided for actuating the pump device (70), and
c. the pump device (70) is designed such that, when the actuator (44) is dis placed from the unactuated end position to the actuated end position, an amount of liquid of less than 70 µl is delivered to the at least one outlet opening (54).
preferably comprising at least one of the following features:
d. the pump device (70) is designed such that, when the actuator (44) is dis placed from the unactuated end position to the actuated end position, an amount of liquid of less than 50 µl, in particular less than 30 µl, is delivered to the at least one outlet opening (54), and/or
e. the unactuated end position and the actuated end position are spaced apart from one another by at least 4 mm, preferably at least 6 mm, and/or
f. the pump device (70) is in the form of a plunger pump with a pump cylinder and a pump plunger, which can be displaced therein to reduce a pump chamber volume, and/or
g. the pump device (70) is designed according to either of Claims 14 and 15.

11. Nasal dispenser (10) according to Claim 10, comprising the following further feature:
a. the pump device (70) and the outlet channel to the outlet opening (54) are designed such that, when an actuating force of 25 newtons is applied, the actuation from the unactuated end position to the actuated end position leads to an average flow of liquid of less than 0.7 ml/second.

12. Nasal dispenser (10) according to one of the preceding claims, comprising the following further feature:
a. the nasal applicator (40) is part of a discharge unit (18) which can be dis placed with respect to the liquid reservoir (12) linearly in an actuation direc tion (6) parallel to the discharge direction (2).

13. Nasal dispenser (10) according to one of the preceding claims, comprising at least one of the following further features:
a. a plurality of inlet channels (96), preferably two opposite inlet channels (96), are provided, and/or
b. the vortex chamber (98) has a rotationally symmetrical inner contour, and/or
c. the vortex chamber (98) is formed by a depression in the inner component (60), wherein the at least one inlet channel (96) is preferably formed by a further depression in the inner component (60) with a smaller depth, and/or
d. the at least one inlet channel (96) has a cross section which tapers in the flow direction (4), with the result that the narrowest point is provided at an opening into the vortex chamber (98).

14. Pump device (70) for a liquid dispenser, in particular a liquid dispenser according to one of the preceding claims, comprising the following features:
a. the pump device (70) has a pump chamber (72) formed by two pump com ponents (74A, 74B) which are movable relative to one another, and
b. the pump device (70) has an inlet valve (76) and an outlet valve (90), which connect the pump device (70) to a liquid reservoir (12) and an outlet open ing (54), and
c. the outlet valve (90) is in the form of a pressure-dependently opening outlet valve (90), and
d. the inlet valve (76) has a valve piston (77) on the first pump component (74A) and a metering channel (78) on the second pump component (74B), which the valve piston (77) enters to close the inlet valve (76) when the pump is being actuated,
**characterized by** the following further features:
e. the pump device (70) has a cylindrical pump chamber wall (80) on the first pump component (74A), and
f. the pump device (70) has a piston surface (82) which bears against the in ner side of the cylindrical pump chamber wall (80), and
g. a sleeve (84), which projects into an inner region of the cylindrical pump chamber wall (80) and the inner side of which forms the metering channel (78) and on the outer side of which and/or at the distal end of which the pis ton surface (82) is provided, is provided on the second pump component (74B).

15. Pump device (70) according to Claim 14, comprising the following further feature:
a. the cross-sectional area of the cylindrical pump chamber wall (80) is be tween 10 mm2 and 20 mm2, preferably between 10 mm² and 17 mm²,
preferably comprising the following additional feature:
b. the cross-sectional area of the metering channel (78) is between 2 mm² and 5 mm², preferably between 3 mm² and 4 mm².

## Revendications

1. Distributeur nasal (10) pour l'application nasale de liquides sous forme atomisée, notamment pour l'application nasale de liquides chez les enfants, avec les caractéristiques suivantes :
a. le distributeur nasal (10) dispose d'un réservoir de liquide (12) pour recevoir du liquide avant la distribution, et
b. le distributeur nasal (10) dispose d'un applicateur nasal (40) pour distribuer le liquide, et
c. un composant extérieur (50) de l'applicateur nasal (40) présente une pointe d'applicateur mince (42) avec une paroi frontale distale (52) qui est traversée par une ouverture de déchargement (54) qui est configurée pour distribuer un jet de pulvérisation dans une direction de déchargement (2), et
d. un composant intérieur (60) de l'applicateur nasal (40) est inséré dans le composant extérieur (50), et
e. le composant extérieur (50) et le composant intérieur (60) délimitent ensemble une chambre de tourbillonnement (98) en amont de l'ouverture de déchargement (54), au moins un canal d'entrée (96) générant des tourbillons étant prévu, qui débouche dans la chambre de tourbillonnement (98),
**caractérisé par** une des caractéristiques supplémentaires suivantes :
f. le composant intérieur (60) et le composant extérieur (50) sont agencés de manière fixe l'un par rapport à l'autre et délimitent ensemble en périphérie l'au moins un canal d'entrée (96), la surface de section transversale du canal d'entrée (96) orientée transversalement à la direction d'écoulement (4) dans le canal d'entrée à son emplacement le plus étroit ou la somme des surfaces de section transversale des canaux d'entrée orientées transversalement à la direction d'écoulement (4) dans les canaux d'entrée (96) à leurs emplacements respectifs les plus étroits étant de 0,05 mm² maximum, de préférence de 0,04 mm² maximum, ou
g. le composant intérieur (60) est agencé de manière mobile dans le composant extérieur (50) et le composant intérieur (60) et le composant extérieur (50) délimitent ensemble l'au moins un canal d'entrée (96), de telle sorte que la section transversale de l'au moins un canal d'entrée (96) est variable en raison de la mobilité du composant intérieur (60) par rapport au composant extérieur (50), et l'ouverture de déchargement (54) présente à son emplacement le plus étroit une surface de section transversale de 0,05 mm² maximum, de préférence de 0,04 mm² maximum.

2. Distributeur nasal (10) selon la revendication 1, avec les caractéristiques supplémentaires suivantes :
a. le composant intérieur (60) et le composant extérieur (50) sont agencés de manière fixe l'un par rapport à l'autre, et
b. l'ouverture de déchargement (54) présente, à son emplacement le plus étroit, une surface de section transversale qui est supérieure à la surface de section transversale du canal d'entrée (96) ou à la somme des surfaces de section transversale des canaux d'entrée (96).

3. Distributeur nasal (10) selon la revendication 2, avec les caractéristiques supplémentaires suivantes :
a. l'ouverture de déchargement (54) présente, à son emplacement le plus étroit, une surface de section transversale d'au moins 0,06 mm²,
de préférence avec la caractéristique supplémentaire suivante :
b. l'ouverture de déchargement (54) présente, à son emplacement le plus étroit, une surface de section transversale d'au moins 0,08 mm².

4. Distributeur nasal (10) selon l'une quelconque des revendications 1 à 3, avec les caractéristiques supplémentaires suivantes :
a. le composant intérieur (60) et le composant extérieur (50) sont agencés de manière fixe l'un par rapport à l'autre, et
b. l'au moins un canal d'entrée (96) est conçu à son emplacement le plus étroit de telle sorte que deux parois de canal opposées (96A, 96B) du canal d'entrée (96), dont l'une est formée par le composant intérieur (60) et l'autre par le composant extérieur (50), définissent une hauteur de canal inférieure à 0,15 mm, de préférence inférieure à 0,12 mm,
de préférence avec la caractéristique supplémentaire suivante :
c. transversalement à la hauteur de canal, l'au moins un canal d'entrée (96) présente, à la section transversale la plus étroite, une largeur de canal qui est supérieure à la hauteur de canal et qui est de préférence au moins deux fois plus large que la hauteur de canal, la largeur de canal étant de préférence d'au moins 0,15 mm, notamment de préférence de 0,18 mm ou plus.

5. Distributeur nasal (10) selon l'une quelconque des revendications 1 à 4, avec les caractéristiques supplémentaires suivantes :
a. le composant intérieur (60) et le composant extérieur (50) sont agencés de manière fixe l'un par rapport à l'autre, et
b. la chambre de tourbillonnement (98) est formée par un renfoncement dans le composant intérieur (60), l'au moins un canal d'entrée (96) étant formé par un autre renfoncement dans le composant intérieur (60) avec une profondeur inférieure.

6. Distributeur nasal (10) selon la revendication 1, avec les caractéristiques supplémentaires suivantes :
a. le composant intérieur (60) est agencé de manière mobile dans le composant extérieur (50), et
b. le composant intérieur (60) et le composant extérieur (50) peuvent être déplacés jusqu'à une position finale dans laquelle un flux entrant dans la chambre de tourbillonnement (98) est empêché,
de préférence avec la caractéristique supplémentaire suivante :
c. le composant intérieur (60) et le composant extérieur (50) forment ensemble une soupape de déchargement commutable par pression de liquide.

7. Distributeur nasal (10) selon la revendication 1 ou 6, avec les caractéristiques supplémentaires suivantes :
a. le composant intérieur (60) est agencé de manière mobile dans le composant extérieur (50), et
b. le composant extérieur (50) présente, sur le côté intérieur de la paroi frontale (52), une nervure (56) entourant l'ouverture de déchargement (54), qui est traversée par au moins un canal d'entrée (96), et
c. le composant intérieur (60) présente un piston de fermeture (62) qui, dans la position finale, recouvre intérieurement l'au moins un canal d'entrée (96) et empêche ainsi un afflux de liquide du canal d'entrée (96) dans la chambre de tourbillonnement (98) ou qui, dans la position finale, s'applique intérieurement par une surface frontale (64) contre le côté intérieur de la paroi frontale (52) du composant extérieur (50).

8. Distributeur nasal (10) selon la revendication 1, 6 ou 7, avec les caractéristiques supplémentaires suivantes :
a. le composant intérieur (60) est agencé de manière mobile dans le composant extérieur (50), et
b. l'ouverture de déchargement (54) présente une section transversale qui varie dans la direction de déchargement (2) depuis un côté intérieur de la paroi frontale (52) jusqu'à un côté extérieur de la paroi frontale (52), un renfoncement ayant une surface de section transversale plus grande par rapport à l'emplacement le plus étroit de l'ouverture de déchargement (54) étant formé sur le côté intérieur et/ou le côté extérieur, notamment un renfoncement cylindrique et/ou conique (58).

9. Distributeur nasal (10) selon l'une quelconque des revendications précédentes, avec la caractéristique supplémentaire suivante :
a. la pointe d'applicateur (42) est symétrique en rotation et est configurée sous forme se rétrécissant vers l'extrémité distale, sa longueur depuis une zone de racine jusqu'à l'extrémité distale étant au moins deux fois plus grande que le diamètre extérieur dans la zone de racine,
de préférence avec la caractéristique supplémentaire suivante :
b. la longueur de la pointe d'applicateur (42) depuis la zone de racine (42A) jusqu'à l'extrémité distale (42B) est d'au moins 15 mm.

10. Distributeur nasal (10) selon l'une quelconque des revendications précédentes, avec les caractéristiques supplémentaires suivantes :
a. le distributeur nasal (10) dispose d'un dispositif de pompage (70) qui est relié par un côté d'entrée au réservoir de liquide (12) et qui est relié par l'intermédiaire d'un canal de déchargement à l'ouverture de déchargement (54), et
b. pour l'actionnement du dispositif de pompage (70), une poignée d'actionnement (44) est prévue, qui peut être déplacée entre une position finale non actionnée et une position finale actionnée, et
c. le dispositif de pompage (70) est conçu de telle sorte que, lors d'un déplacement de la poignée d'actionnement (44) de la position finale non actionnée à la position finale actionnée, une quantité de liquide inférieure à 70 µl est transportée vers l'au moins une ouverture de déchargement (54),
de préférence avec au moins une des caractéristiques suivantes :
d. le dispositif de pompage (70) est conçu de telle sorte que, lors d'un déplacement de la poignée d'actionnement (44) de la position finale non actionnée à la position finale actionnée, une quantité de liquide inférieure à 50 µl est transportée vers l'au moins une ouverture de déchargement (54), notamment inférieure à 30 µl, et/ou
e. la position finale non actionnée et la position finale actionnée sont espacées l'une de l'autre d'au moins 4 mm, de préférence d'au moins 6 mm, et/ou
f. le dispositif de pompage (70) est configuré sous forme de pompe à piston avec un cylindre de pompe et un piston de pompe pouvant être déplacé dans celui-ci pour réduire un volume de chambre de pompe et/ou
g. le dispositif de pompage (70) est configuré selon l'une quelconque des revendications 14 ou 15.

11. Distributeur nasal (10) selon la revendication 10, avec la caractéristique supplémentaire suivante :
a. le dispositif de pompage (70) et le canal de déchargement vers l'ouverture de déchargement (54) sont conçus de telle sorte que, pour une force d'actionnement de 25 Newton, l'actionnement depuis la position finale non actionnée jusqu'à la position finale actionnée entraîne un débit de liquide moyen inférieur à 0,7 ml/seconde.

12. Distributeur nasal (10) selon l'une quelconque des revendications précédentes, avec la caractéristique supplémentaire suivante :
a. l'applicateur nasal (40) fait partie d'une unité de déchargement (18) pouvant être déplacée de manière linéaire par rapport au réservoir de liquide (12) dans une direction d'actionnement (6) parallèle à la direction de déchargement (2).

13. Distributeur nasal (10) selon l'une quelconque des revendications précédentes, avec au moins une des caractéristiques supplémentaires suivantes :
a. il est prévu une pluralité de canaux d'entrée (96), de préférence deux canaux d'entrée (96) opposés l'un à l'autre, et/ou
b. la chambre de tourbillonnement (98) présente un contour intérieur symétrique en rotation, et/ou
c. la chambre de tourbillonnement (98) est formée par un renfoncement dans le composant intérieur (60), l'au moins un canal d'entrée (96) étant de préférence formé par un autre renfoncement dans le composant intérieur (60) avec une profondeur plus faible, et/ou
d. l'au moins un canal d'entrée (96) présente une section transversale qui se rétrécit dans la direction d'écoulement (4), de telle sorte que l'emplacement le plus étroit est prévu à une embouchure dans la chambre de tourbillonnement (98).

14. Dispositif de pompage (70) pour un distributeur de liquide, notamment un distributeur de liquide selon l'une quelconque des revendications précédentes, avec les caractéristiques suivantes :
a. le dispositif de pompage (70) présente une chambre de pompe (72) formée par deux composants de pompe (74A, 74B) mobiles l'un par rapport à l'autre, et
b. le dispositif de pompage (70) présente une soupape d'admission (76) et une soupape d'échappement (90) qui relient le dispositif de pompage (70) à un réservoir de liquide (12) et à une ouverture de déchargement (54), et
c. la soupape d'échappement (90) est configurée sous forme de soupape d'échappement (90) s'ouvrant en fonction de la pression, et
d. la soupape d'admission (76) présente un piston de soupape (77) sur le premier composant de pompe (74A) et un canal de dosage (78) sur le deuxième composant de pompe (74B), dans lequel le piston de soupape (77) pénètre pour fermer la soupape d'admission (76) au cours d'un actionnement de la pompe,
**caractérisé par** les caractéristiques supplémentaires suivantes :
e. le dispositif de pompage (70) présente une paroi de chambre de pompe cylindrique (80) sur le premier composant de pompe (74A), et
f. le dispositif de pompage (70) présente une surface de piston (82) s'appliquant intérieurement contre la paroi de chambre de pompe cylindrique (80), et
g. sur le deuxième composant de pompe (74B) est prévu un manchon (84) qui pénètre dans une zone intérieure de la paroi de chambre de pompe cylindrique (80) et dont le côté intérieur forme le canal de dosage (78) et sur le côté extérieur et/ou à l'extrémité distale duquel est prévue la surface de piston (82).

15. Dispositif de pompage (70) selon la revendication 14, avec la caractéristique supplémentaire suivante :
a. la surface de section transversale de la paroi de chambre de pompe cylindrique (80) est comprise entre 10 mm² et 20 mm², de préférence entre 10 mm² et 17 mm²,
de préférence avec la caractéristique supplémentaire suivante :
b. la surface de section transversale du canal de dosage (78) est comprise entre 2 mm² et 5 mm², de préférence entre 3 mm² et 4 mm².
